# EUROPÄISCHE PATENTSCHRIFT

(11) **EP 1 106 206 B1**
(45) Veröffentlichungstag und Bekanntmachung des Hinweises auf die Patenterteilung: **27.06.2007**
(21) Anmeldenummer: 00250411.6
(22) Anmeldetag: 30.11.2000
(51) Int. Cl.: A61N 1/368, A61N 1/362, A61N 1/365, A61N 1/37, A61N 1/39

(54) **Gerät zur Regelung der Herzfrequenz und der Herzpumpkraft**
Apparatus for controlling the rate and the pumping action of the heart
Appareil de régulation de la fréquence et de l'action de pompage du coeur

(30) Priorität: 30.11.1999 DE 19957649
(43) Veröffentlichungstag der Anmeldung: 13.06.2001
(73) Patentinhaber: BIOTRONIK GmbH & Co. KG, 12359 Berlin (DE)
(72) Erfinder: Schauerte, Patrick, 52134 Herzogenrath (DE); Schaldach, Max, 91054 Erlangen (DE)
(74) Vertreter: Lindner-Vogt, Karin L.

(56) Entgegenhaltungen:
- EP-A- 0 688 579
- EP-A- 0 879 621
- US-A- 5 318 592

## Beschreibung

Die Erfindung betrifft ein Gerät zur Therapie supraventrikulärer und ventrikulärer bradykarder und tachykarder Rythmusstörungen und/oder zur Beeinflussung der Herzpumpkraft

Die normale Herzfrequenz beim Menschen liegt in Ruhe zwischen 60 und 100 Schläge/min, unter Belastung kann sie auf bis zu 180/min ansteigen. Eine Ruheherzfrequenz, die unter 60 /min liegt, wird im allgemeinen als Bradykardie bezeichnet, während eine Ruheherzfrequenz, die über 100/min liegt, als Tachykardie bezeichnet wird. Tachykardien können in den Herzvorhöfen ihren Ursprung nehmen (sogenannte supraventrikuläre Tachykardien und Vorhofflimmern) oder in den Herzhauptkammern entstehen (sogenannte ventrikuläre Tachykardien und Kammerflimmern). Bradykardien können einerseits Ausdruck einer verlangsamten Impulsgebung im normalen Schrittmacherzentrum des Herzens sein (dem sogenannten Sinusknoten) oder aber durch eine pathologische Überleitungsverzögerung der Erregung von den Vorhöfen auf die Herzhauptkammern verursacht werden (sogenannte AV-Knotenerkrankung). Die bei weitem häufigste supraventrikuläre Tachykardie, das Vorhofflimmern tritt mit zunehmendem Lebensalter vermehrt auf und ist bei mehr als 5 % der Menschen im Alter über 65 Jahre vorhanden. Eine supraventrikuläre Tachykardie führt vor allem über eine Verminderung der Diastolenzeit zu einer verminderten ventrikulären diastolischen Füllung des Herzens und damit zu einem verminderten Herzzeitvolumen. Dies führt vor allem bei vorbestehender Herzpumpschwäche (sogenannter Herzinsuffizienz) zu arterieller Hypotonie (Vorwärtsversagen) und zu einem Rückstau von Blut in den Lungen, was die Sauerstoffanreicherung des Blutes in den Lungen beeinträchtigt (sogenanntes Rückwärtsversagen). Darüber hinaus steigt der Sauerstoffverbrauch des Herzens bei gleichzeitig verminderter diastolischer Koronardurchblutung.

Es sind Stimulationsgeräte bekannt, welche das Herzverhalten durch Stimulation und oder Tachycarditerminierung bzw. Cardioversion beeinflussen. Diese Geräte weisen jedoch ausschließlich Erfassungsgeräte und Stimulationseinrichtungen auf, welche auf bestimmte Eingangskriterien hin Signale auslösen, welche ausschließlich zur direkt auf das mechanische Verhalten des Herzens durch Stimulation von Bereichen, des Herzens, auf denen sich die mit der Kontraktion des Herzens in unmittelbarer Verbindung stehenden elektrischen Aktivtätssignale ausbreiten.
Dies ist nachteilig, weil hiermit Signalereignisse, welche mit diesen unmittelbar das Herzgeschehen beeinflussenden Signalen in Verbindung stehen nicht beeinflusst werden.

Aus EP 0 688 579 ist ein Herztherapiegerät bekannt, welches neben einem Tachyarrithmiedetektor einen Stromgenerator aufweist, mit dessen Hilfe über ein Elektrodensystem Stromimpulse an einem zum parasympathischen Nervensystem gehörenden Nerv abgegeben werden können, falls eine Tachyarrithmie erfasst wird.

Der Erfindung liegt die Aufgabe zugrunde ein Gerät er eingangs genannten Gattung zu schaffen, welches geeignet ist, schon im Vorfeld der bekannten Erzeugung von Stimulations- bzw. Defibrillations- oder Cardioversionssignalen regelnd einzugreifen.

Diese Aufgabe ist mit den im Anspruch 1 angegebenen Maßnahmen gelöst.

Die Erfindung beruht unter anderem auf der Erkenntnis, dass die Frequenz supraventrikulärer Tachykardien durch Aktivierung des parasympathischen autonomen Nervensystems gesenkt und durch Aktivierung des sympathischen Nervensystems gesteigert werden kann. Entsprechend kann auch die Herzleistung insgesamt gesteigert und/oder vermindert werden. Eine frequenzsteigernde/senkende Wirkung auf den Sinusknoten wird als positiv/negativ chronotrope Wirkung bezeichnet, während eine die atrioventrikuläre Überleitung (AV-Überleitung) fördernde/hemmende Wirkung als positiv/negativ dromotrope Wirkung bezeichnet wird.

Parasympathische Nervenfasern, die den Sinusknoten, die Vorhöfe und den atrioventrikulären Knoten innervieren, verlaufen entlang der oberen Hohlvene, des Koronarsinus und der rechten Pulmonalarterie. Sympathische Nervenfasern, die zu einem Anstieg der Sinusknoten und/oder Vorhoffrequenz und zu einer Beschleunigung der atrioventrikulären Überleitung führen, verlaufen vom stellatum ganglion über eine dorsale, sich von hinten an die arteria subclavia anlegende Nervenschlinge (sogenannte dorsale ansa subclavia) und eine ventrale, sich von vorne an die arteria subclavia anlegende Nervenschlinge (sogenannte ventrale ansa subclavia) zum Herzmuskel. Die ansae subclaviae enthalten nahezu alle sympathischen Nervenfasern, die vom ganglion stellatum zum Herzmuskel führen. Dabei handelt es sich überwiegend um präganglionäre Nervenfasern, die im ganglion cervikale medius und den oberen, thorakalen Grenzstrangganglien auf postganglionäre Fasern umgeschaltet werden.

Ventrikuläre Tachykardien weisen ähnliche hämodynamische Veränderungen auf wie supraventrikuläre Tachykardien. Da jedoch im Gegensatz zu vielen supraventrikulären Tachykardien eine synchronisierte Vorhofkontraktion vor der Kammerkontraktion fehlt, ist bei gleicher Frequenz eine ventrikuläre Tachykardie hämodynamisch ungünstiger als eine supraventrikuläre Tachykardie. Vor allem aber der veränderte intraventrikuläre Kontraktionsablauf bei ventrikulären Tachykardien führt zu einer deutlichen Schlagvolumenminderung und arteriellen Hypotonie, was dazu führt, daß Patienten mit einer ventrikulären Tachykardie in der Regel schneller bewußtlos werden als Patienten mit einer supraventrikulären Tachykardie gleicher Frequenz. Vor allem aber degenerieren ventrikuläre Tachykardien schnell in Kammerflimmern, einem Zustand, in dem die Herzhauptkammern asynchron und unvollständig mit Frequenzen > 400 Schläge/min schlagen. Dies führt zu einem Verlust eines arteriellen Blutdruckes. Ventrikuläre Tachykardien und Kammerflimmern sind die Hauptursache des sogenannten plötzlichen Herztodes, der für ca. 80% der kardial bedingten Todesfälle jährlich verantwortlich ist.

Das sympathische, autonome Nervensystem spielt eine Schlüsselrolle bei der Entstehung von ventrikulären Tachykardien und Kammerflimmern. So können die sympathischen Neurotransmitter Adrenalin, Noradrenalin abnorme Automatizität und ventrikuläre Extrasystolen im Infarktgebiet auslösen oder die Leitungsgeschwindigkeit durch Myokardnarben nach einem Herzinfarkt beschleunigen, was die Entstehung von ventrikulären Kreisbahnen und ventrikulären Tachykardien begünstigt.

Schließlich finden sich bei Patienten nach Herzinfarkten häufig Herzmuskelbezirke, in denen auch die sympathischen Herznerven zugrunde gegangen sind, was eine Denervierungsüberempfindlichkeit dieser Areale auf Adrenalin und Noradrenalin verursacht. Eine solche Überempfindlichkeit und inhomogene sympathische Innervierung begünstigt die Entstehung von ventrikulären Tachykardien und Kammerflimmern.

Das parasympathische, autonome Nervensystem und sein Neurotransmitter Acetylcholin antagonisieren den Einfluß des sympathischen Nervensystems auf das Herz und können den plötzlichen Herztod in Tiermodellen verhindern.

Klinische Testverfahren, die den sympathischen und parasympathischen Tonus bei Patienten messen, haben gezeigt, daß ein erhöhter Sympathikotonus und erniedrigter Parasympathikotonus das Auftreten eines plötzlichen Herztodes signifikant begünstigen.

Parasympathische Nervenfasern, die die Herzhauptkammern innervieren, akkumulieren in einem Fettbürzel in der Höhe des Koronarsinus im Bereich der proximalen linken Herzkranzarterie.

Die zweite wesentliche Einflußgröße auf das Herzzeitvolumen ist neben der Herzfrequenz die Kontraktionskraft des Herzens. Sie beschreibt, welche Menge Blut pro Herzschlag (Schlagvolumen) ausgeworfen wird. Darüber hinaus bestimmt sie das Ausmaß und die Geschwindigkeit des Druckanstieges in der Schlagader bei einem Herzschlag. Zahlreiche Erkrankungen, die zu einem Untergang von Herzmuskulatur führen, wie zum Beispiel eine koronare Herzerkrankung mit Herzinfarkten, können zu einer Verminderung der Pumpkraft des Herzens führen. Dies führt dazu, daß bei normaler Herzfrequenz die Pumpkraft des Herzmuskels nicht ausreicht, um ein minimal notwendiges Schlagvolumen zurAufrechterhaltung eines normalen arteriellen Blutdruckes und zur Verhinderung des Blutstaus vor dem Herzens zu ermöglichen. Einflußgrößen, die zu einer Steigerung der Kontraktionskraft des Herzens führen, werden als positiv inotrope Größen bezeichnet. Positiv inotrope Wirkungen besitzen vor allem Katecholamine wie Adrenalin und Noradrenalin, die vom sogenannten sympathischen autonomen Nervensystem als Neurotransmitter ausgeschüttet werden.

Sympathische Nervenfasern, die die Herzhauptkammern innervieren, verlaufen in einer ventralen und dorsalen Nervenschlinge um die rechte und linke arteria subclavia. Weitere sympathische Nervenfasern ziehen in einem ventrolateralen Nerv vom stellatum ganglion bzw. dem inferioren cervikalen Grenzstrangganglion entlang der Pulmonalvenen und dem Koronarsinus zu den Herzhauptkammern.

Gemäß der vorliegenden Erfindung wird - einschlließlich vorteilhafter Weiterbildungen - ein medizinisches Elektrostimulationsgerät zur Therapie supraventrikulärer und ventrikulärer bradykarder und tachykarder Rhythmusstörungen und zur Steigerung der Herzpumpkraft geschaffen mit
- Elektroden zur elektrischen und/oder magnetischen Stimulation parasympathischer Nerven, die den Sinusknoten, die Vorhöfe, den atrioventrikulären Knoten oder die Herzhauptkammern innervieren;
- Elektroden zur elektrischen und/oder magnetischen Stimulation der Vorhöfe und Herzhauptkammern sowiezurventrikulären Kardioversion/Defibrillation;
- einer Einrichtung zur Erzeugung von elektrischen und/oder magnetischen Stimulationsimpulsen, die zu den Elektroden geleitet werden;
- einer Einrichtung zur Erfassung der Rate, mit der die menschlichen Herzvorhöfe und Herzhauptkammern schlagen, wobei diese Einrichtung atriale und ventrikuläre Depolarisationen mißt;
- einer Einrichtung zur Erfassung biologischer Parameter wie des arteriellen Blutdruckes, des rechts- oder linksventrikulären Druckes, der Sauerstoffsättigung des Blutes oder des Herzzeitvolumens; der myokardialen oder thorakalen Impedanz oder von monophasischen Aktionspotentialen oder evozierten myokardialen Potentialen;
- einer Einrichtung zur Programmierung einer Frequenzgrenze, oberhalb oder unterhalb der eine Schlagrate des Herzens als Tachykardie oder Bradykardie erkannt wird;
- einer Einrichtung zur Programmierung einer Blutdruck/Herzzeitvolumengrenze, unterhalb der eine behandlungsbedürftige Herzinsuffizienz erkannt wird;
- einer Einrichtung zur Programmierung einer Blutdruck/Herzzeitvolumengrenze, oberhalb der eine sympathische Nervenstimulation inhibiert oder reduziert wird;
- eine auf die Erfassungseinheit reagierende Starteinheit, die die Stimulationsimpulse erzeugende Einrichtung aktiviert, wenn die erfaßte Herzschlagrate der Vorhöfe oder Herzhauptkammern die programmierte Frequenzgrenze überschreitet/unterschreitet;
- eine auf die Erfassungseinheit reagierende Starteinheit, die die Stimulationsimpulse erzeugende Einrichtung aktiviert, wenn der arterielle Blutdruck/Herzzeitvolumen eine programmierte untere Grenze unterschreitet.
- Die Stimulationsimpulse können über einen definierten Zeitraum kontinuierlich oder als kurze Salven abgegeben werden. Stimulationssalven wiederum können entweder asynchron oder sanchronisiert zur atrialen oder ventrikulären Depolarisation abgegeben werden. Die Synchronisation erfolgt dann mit unterschiedlicher zeitlicher Verzögerung zurgemessenen atrialen/ventrikulären Depolarisation in der atrialen/ventrikulären Refraktärzeit;
- einer Einheit, die die während der Stimulation von den Erfassungseinheiten gemessene Vorhof-/Herzkamerfrequenz sowie den arteriellen Blutdruck und das Herzzeitvolumen mit den korrespondierenden Werten vor/ohne Stimulation sowie den entsprechenden programmierten Grenzwerten vergleicht;
- eine Stimulationseinheit, die atrial und/oder ventrikulär elektrisch und/oder magnetisch myokardial stimulieren oder kardiovertieren/defibrillieren kann.

Zur elektrischen und/oder magnetischen Stimulation parasympathischer Nervenfasern, die die Vorhoffrequenz bei supraventrikulären Tachykardien senken soll und/oder die Kammerfrequenz bei supraventrikulären Tachykardien verlangsamen soll, werden Elektroden in der vena cava superior, der vena cava inferior, im rechten Vorhof, im Koronarsinus, in beiden Jugularvenen, der rechten oder linken vena anonyma oder der Pulmonalarterie implantiert.

Zur elektrischen und/oder magnetischen Stimulation parasympathischer Nervenfasern, die vertrikuläre Tachykardien/Kammerflimmern verhindern oder beenden sollen, werden Elektroden im Koronarsinus implantiert.

Zur elektrischen und/oder magnetischen Stimulation parasympathischer Nervenfasern, die die Sinusknoten innervieren werden Elektroden in den Jugularvenen, der oberen Hohlvene, dem lateralen rechten Vorhof im Bereich der Kreuzung der Pulmonalvenen oder in den Pulmonararterien implantiert.

Zur elektrischen und/oder magnetischen Stimulation sympathischer Nervenfasern, die die Herzfrequenz oder die Pumpkraft des Herzens steigern soll, werden Elektroden in der rechten und/oder linken arteria subclavia oder vena subclavia oder den Pulmonalvenen oder dem Koronarsinus implantiert.

Die Stimulationselektroden können intravaskulär oder extravaskulär/epi-kardial an den bezeichneten Stimulationsorten befestigt werden. Eine Nervenstimulation kann unipolar oder bipolar erfolgen, wobei die bipolare Referenzelektrode Teil der Nervenstimulationselektrode sein kann oder Teil einer zweiten in der Nähe der ersten Nervenstimulationselektrode implantierten Nervenstimualtionselektrode sein kann. Die Fixierung der Sonden kann dabei aktiv, zum Beispiel durch Schraubmechanismen oder passiv, z.B. durch Ankervorrichtungen erfolgen.

Die Impulserzeugungseinrichtung zur Stimulation autonomer Nerven sowie zur elektrischen und/oder magnetischen myokardialen Stimulation kann jede geeignete Technologie zum Erzeugen von Stimulationsimpulsen einer Frequenz von 0 bis 100 Hz und einer Einzelimpulsdauer von 0 bis 10 ms aufweisen. Die Impulse können mophasisch oder biphasisch sein.

Durch eine Modifikation der Impulserzeugungseinheit können anstelle von oder zusätzlich zu elektrischen Impulsen auch magnetische Wechselfelder im Pico- bis µ-Tesla-Bereich erzeugt werden, die über entsprechende Elektroden, welche entsprechende Spulenstrukturen aufweisen and die Nerven und/oder das Myocard abgegeben werden.

Die Nervenstimulation wird typischerweise mit 20 Hz bei einer Impulsdauer von 0,1 bis 4 ms durchgeführt.

Bei Vorliegen einer ventrikulären Tachykardie werden kurze Salven (typischerweise 10 bis 50 ms Dauer) hochfrequenter Einzelimpulse unmittelbar nach der ventrikulären Depolarisation (R-Zacke) abgegeben, um eine ventrikuläre myokardiale Depolarisation während Stimulation der parasympathischen Nerven entlang des Koronarsinus zu vermeiden. Ebenso erfolgt die Stimulation des ventrolateralen Nerven, der nah am atrialen/ven-trikulären Myokard verläuft, vorzugsweise in Salven (typischerweise 10 bis 50 ms Dauer) hochfrequenter Einzelimpulse, die unmittelbar nach der ventrikulären Depolarisation (R-Zacke) abgegeben werden, um eine ventrikuläre, myokardiale Depolarisation während Nervenstimulation zu vermeiden.

Die Elektroden zur Erfassung der atrialen/ventrikulären Frequenz befinden sich im Vorhof/Ventrikel und sind mit einem regelbaren Signalverstärker verbunden, der das erfaßte Signal je nach Signalgröße unterschiedlich stark verstärkt. Die Bandpasseigenschaften des Filters dieses Verstärkers sind zum Erfassen von Vorhof-/Ventrikeldepolarisationen optimiert. Die Arbeitsweise des Verstärkers/Filters kann der von bekannten Vorhof-/Kam-merschrittmachern entsprechen.

Mit den erfinderischen Maßnahmen ist es möglich durch Vergleich der aktuellen Herzrate mit einer über einen physiologischen Parameter (Aktivitätsparameter) für den Herzleistungsbedarf durch sympathische bzw. parasympathische Stimulation - je nachdem ob die aktuelle Herzleistung den aktuellen Bedarf gerade über- oder unterschreitet - die Herzrate mittelbar zu beeinflussen. Dies erfolgt im Gegensatz zu bisherigen ratengesteuerten Herzschrittmachern, bei denen die gewünschte Stimulationsrate durch unmittelbare Stimulation des Herzmuskels mit der entsprechenden Rate eingestellt wurde.

Andere günstige Weiterbildungen sind in den Unteransprüchen gekennzeichnet. Ein vorteilhaftes Ausführungsbeispiel der wird nachfolgend anhand der Zeichnungen näher beschrieben. Es zeigen:
- Fig. 1: ein Ausführungsbeispiel einer vorteilhaften Stimulationseinrichtung als Blockschaltbild;
- Fig. 2: ein Zeitdiagramm als Beispiel für die parasympathische Stimulation in der oberen Hohlvene zur Senkung der Vorhoffrequenz bei supraventrikulären Tachykardien;
- Fig. 3: ein Zeitdiagramm als Beispiel der parasympathischen Stimulation im Koronarsinus oder der rechten und/oder linken arteria/vena jugularis zur Terminierung/Verhinderung von ventrikulären Tachykardien;
- Fig. 4: ein weiteres Zeitdiagramm als Beispiel der sympathischen Stimulation in der linken arteria subclavia;
- Fig. 5: ein weiteres Zeitdiagramm als Beispiel die sympathischen Stimulation der ansa subclavia zur Verhinderung einer neurokardiogenen Synkope sowie
- Fig. 6: eine schematische Übersicht über die Anbringungsorte der geannten Elektroden bei einer Schnittdarstellung des Herzens.

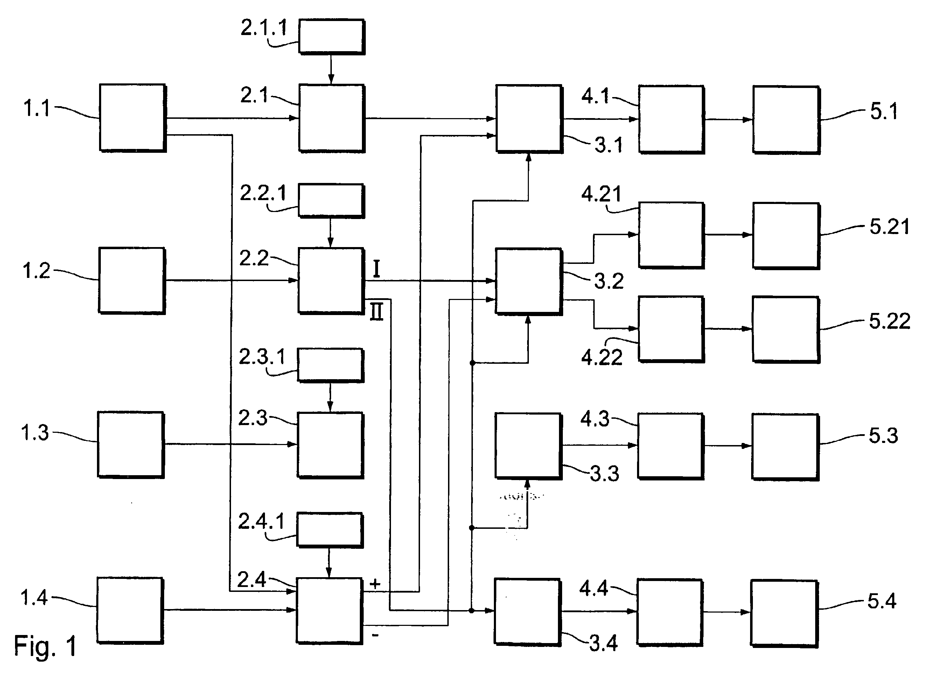

Bei dem in Fig. 1 dargestellten Blockschaltbild ist ein vorteilhaftes Ausführungsbeispiel systematisiert wiedergegeben. In der horizontalen Richtung sind jeweils nacheinander die Erfassungseinheiten, bestehend aus Sensor- (1.x) und Diskriminatorteilen (2.x), die Ansteuerung einer Starteinheit (3.x) zur Aktivierung der entsprechenden Impulserzeugereinheit (4.x) über nachgeschaltete Elektroden (5.x) dargestellt. Eine Programmiereinheit (2.x.1) dient jeweils zur Voreinstellung von Grenzwerten, bei denen die jeweiigen Starteinheiten ansprechen.

Die Zeilen beziehen sich in ihrem linken Teil auf die erfassten Signale und in ihrem rechten Teil auf die einzuleitenden Stimulationsmaßnahmen und die entsprechenden Elektroden sowie die zugehörigen Start- und Stimulationseinheiten.

Die erste Zeile (x.1) bezieht sich auf die Beseitung von Herzinsuffizienz durch sympathische Stimulation mit der entsprechenden Erfassung einer Herzleistung durch den Sensor 1.1 und Vergleich mit einem vorprogrammierten Grenzwert.

In der zweiten Zeile (x.1) erfolgt eine Ratenerkennung (AV) im Hinblick auf die Herztätigkeit ohne Stimulation. Hierdurch wird auf eine Tachycardieerkennung hin, je nach Art der Tachycardie (supraventrikulär, ventrikulär), über unterschiedliche Impulserzeuereinheiten (4.21 und 4.22 in unterschiedlichen Bereichen über entsprechend angeordnete Elektroden (5.21 bzw. 5.22) parasympathisch stimuliert. Im Diskriminatorteil 1.3 wird die letzte Herzrate im nicht stimulierten Betrieb auch zum Vergleich mit dem Ergebnis bei nachfolgender parasympathischer oder sympathischer Stimulation zwecks Erfolgskontrolle festgehalten.

In der dritten Zeile erfolgt die übliche Bradycardieerkennung im Rahmen der normalen Schrittmachertherapie, mit Escape-Intervallen und die entsprechende Steuerung der im Atrium und/oder Vorhof gelegenen Elektroden in konventioneller Herzschrittmachertechnik.

In der vierten Zeile wird über eine physiologische Sensorgröße durch den Sensor 1.4 ein Sollwert für den aktuellen Herzleistungsbedarf ermittelt. Hierbei kann es sich um jede Größe handeln, die bisher bei ratengesteuerten Schrittmachern zur Beeinflussung der Herzrate verwendet wurde. Durch Vergleich mit der Ausgangsgröße des Sensors 1.1 für die Herzleistung und eine vorprogrammierte Abhängigkeit bis zu ebenfalls vorprogrammierten Grenzwerten (die Sollfunktion der Herzrate in Abhängigkeit vom Herzleistungsbedarf) wird bei zu geringer Herztätigkeit eine sympathische Stimulation Blöcke 4.1, 5.1 eine sympathische Stimulation und im Falle zu großer Herztätigkeit eine parasympathische Stimulation eingeleitet (Blöcke 4.21 und 5.21 bzw. 4.22 und 5.22). Auf diese Weise ist erstmalig eine echte "Regelung" der Herzfrequenz durch mittelbare Stimulation möglich.

Für die Signalzuordnungen und Definitionen wird - zur Vermeidung von Wiederholungen - auf die übrigen Beschreibungsteile und die Ansprüche verwiesen.

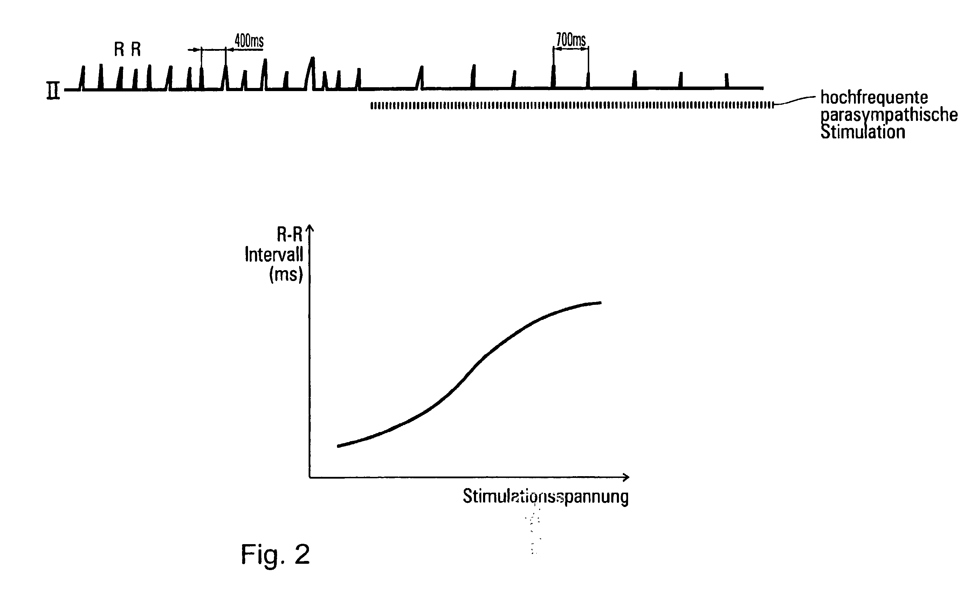

Fig. 2 zeigt ein Beispiel der parasympathischen Stimulation in der oberen Hohlvene zur Senkung der Vorhoffrequenz bei supraventrikulären Tachykardien sowie Senkung der Kammerfrequenz bei Vorhofflimmern. Übersteigt die Vorhoffrequenz (Kammerfrequenz bei Vorhofflimmern) eine programmierte obere Grenzfrequenz, wird über die Starteinheit eine Salve von hochfrequenten elektrischen und/oder magnetischen Impulsen (typischerweise 20 Hz, Impulsdauer 2 ms, Impulsspannung 5 bis 20 V) initiiert. Diese Impulse werden über die in/an der oberen Hohlvene positionierte Nervenstimulationselektrode abgegeben. Während der parasympathischen Stimulation wird durch eine Vergleichseinheit die Vorhoffrequenz/Kammerfrequenz während parasympathischer Stimulation mit der programmierten oberen Frequenzgrenze verglichen. Wird diese obere Frequenzgrenze nicht mit der initialen Nervenstimulationsintensität unterschritten, erfolgt die stufenweise Erhöhung der Nerverstimualtionsintensität, bis die Vorhof-/Kammerfrequenz die programmierte obere Grenzfrequenz unterschreitet. Bei Unterschreiten der oberen Grenzfrequenz während parasympathischer Stimulation erfolgt die automatische Senkung der Nervenstimulationsintensität. Die obere Grenze der Vorhof-/Kammerfrequenz bei Vorhoftachykardien/Vorhof-flimmern kann automatisch an die körperliche Aktivität des Patienten angepaßt werden. Sensoren zur Messung der körperlichen Aktivität können denen von bekannten Vorhof-/Kammerschrittmachem entsprechen.

Kommt es während parasympathischer Stimulation in der oberen Hohlvene zu einem Abfall des Herzzeitvolumens/arteriellen Blutdruckes unter einen festgesetzten Grenzwert, wird die parasympathische Stimulationsintensität in der oberen Hohlvene reduziert, bis der untere Grenzwert des Herzzeitvolumens/arteriellen Blutdruckes wieder überschritten ist.

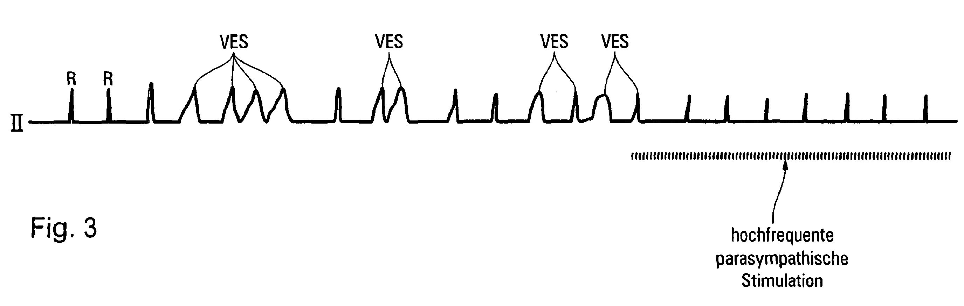

Fig. 3 zeigt ein Beispiel der parasympathischen Stimulation im Koronarsinus oder der rechten und/oder linken arteria/vena jugularis zur Terminierung/Verhinderung von ventrikulären Tachykardien. Das Auftreten von gehäuften ventrikulären Extrasystolen oder nicht anhaltenden ventrikulären Salven (Dauer < 10 Sekunden) löst über die Starteinheit eine Salve von hochfrequenten, elektrischen und/oder magnetischen Impulsen (typischerweise 20 Hz, Impulsdauer 2 ms, Impulsspannung 5 bis 20 V) aus. Diese Impulse werden über die im/am Koronarsinus positionierten Nervenstimulations-elektroden abgegeben. Die Anzahl und Vorzeitigkeit der ventrikulären Extrasystolen bzw. die Zykluslänge der nicht anhaltenden, ventrikulären Tachykardien, die eine parasympathische Stimulation auslösen, kann frei programmiert werden. Während der parasympathischen Stimulation wird durch eine Vergleichseinheit die Kammerfrequenz und das Auftreten von ventrikulären Extrasystolen sowie nicht anhaltender ventrikulärer Salven während parasympathischer Stimulation mit den programmierten Grenzwerten verglichen. Werden die programmierten Grenzwerte während parasympathischer Stimulation über-/unterschritten, erfolgt die stufenweise Erhöhung/Verminderung der Nervenstimulationsintensität, bis die programmierten Grenzwerte der ventrikulären Extrasystolen/nicht anhaltenden, ventrikulären Tachykardien unterschritten werden.

Kommt es während parasympathischer Stimulation im Koronarsinus/vena/arteria jugularis zu einem Abfall des Herzzeitvolumens/arteriellen Blutdruckes unter einen festgesetzten Grenzwert, wird die parasympathische Stimulationsintensität im Koronarvenensinus reduziert, bis der untere Grenzwert des Herzzeitvolumens/arteriellen Blutdruckes wieder überschritten ist.

Im Falle einer ventrikulären Tachykardie erfolgt die parasympathische Stimulation im Koronarsinus/vena jugularis interna/externa, wobei die Stimulationssalven synchronisiert auf die R-Zacken in der ventrikulären Refraktärzeit abgegeben werden, um eine ventrikuläre myokardiale Stimulation zu vermeiden. Die Diagnose einer ventrikulären Tachykardie basiert auf zwei Kriterien: Die Herzhauptkammerfrequenz überschreitet eine festgesetzte Grenzfrequenz und es kommt zu keinem Abfall der Kammerfrequenz während probatorischer parasympathischer Stimulation in der oberen Hohlvene. Kommt es unter parasympathischer Stimulation nicht zu einer Terminierung der ventrikulären Tachykardie wird ein ventrikulärer antitachykarder Überstimulationsversuch oder eine Kardioversion initiiert. Vor und während der Kardioversion/Defibrillation wird die parasympathische Stimulation im Koronarvenensinus oder der rechten und/oder linken vena jugularis interna fortgesetzt. Hierdurch läßt sich die Energiemenge senken, die zur ventrikulären Kardioversion benötigt wird. Auch bei der Defibrillation von Kammerflimmern kann die parasympathische Stimulation, Stimulation im Koronarvenensinus oder der rechten und/oder linken vena jugularis interna initiiert werden, um die ventrikuläre Defibrillationsschwelle zu senken. Analog kann auch eine parasympathische Stimulation in der rechten/linken vena/arteria jugularis, der oberen Hohlvene, dem Koronarvensinus oder der Pulmonalarterie unmittelbar vor und während einer Kardioversion von Vorhofflimmern abgegeben werden, um die Artikel Kardioversionsschwelle zu senken.

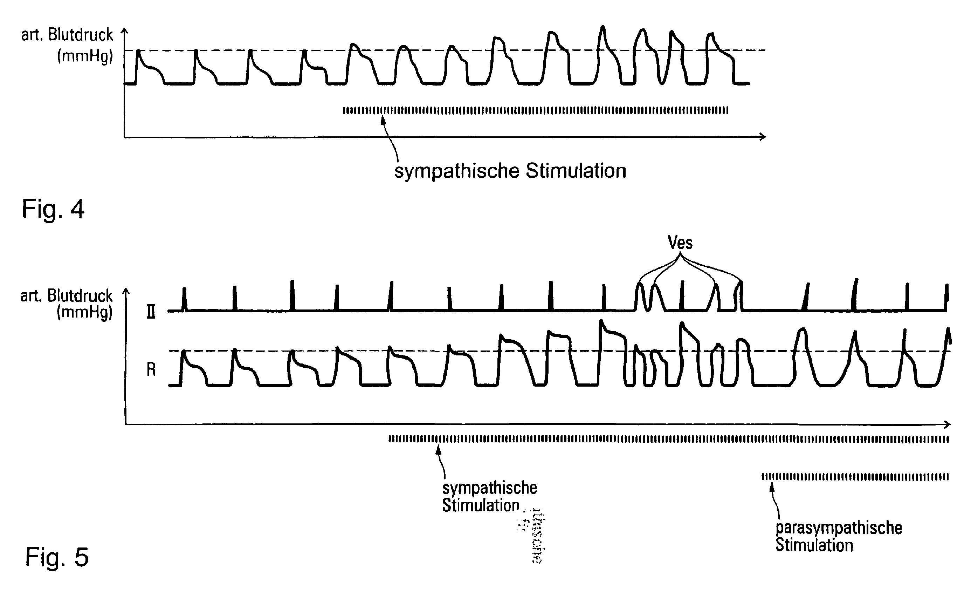

Fig. 4 verdeutlicht ein Beispiel der sympathischen Stimulation in der linken arteria subclavia. Fällt der arterielle Blutdruck/Herzzeitvolumen unter einen programmierten Grenzwert, erfolgt die sympathische Stimulation, bis dieser Grenzwert überschritten wird. Übersteigt die Vorhoffrequenz oder Kammerfrequenz bei sympathischer Stimulierung einen programmierten oberen Grenzwert, wird die Stimulationsintensität verringert bis die Herzfrequenz unter diesen Grenzwert fällt. Die sympathische Stimulationsintensität wird in diesem Fall nur soweit reduziert als der arterielle Blutdruck/Herzzeitvolumen oberhalb des entsprechenden programmierten Grenzwertes bleibt. Sollte während der Reduktion der sympathischen Nervenstimulationsintensität der arterielle Blutdruck/Herzzeitvolumen unter den entsprechenden Grenzwert fallen, erfolgt erneut eine Erhöhung der sympathischen Stimulationsintensität. Gleichzeitig wird durch die Starteinheit die parasymathische Stimulation in der oberen Hohlvene initiiert, bis die Vorhoffrequenz/Kammerfrequenz der Jugularvenen unter die entsprechende Grenzfrequenz fällt.

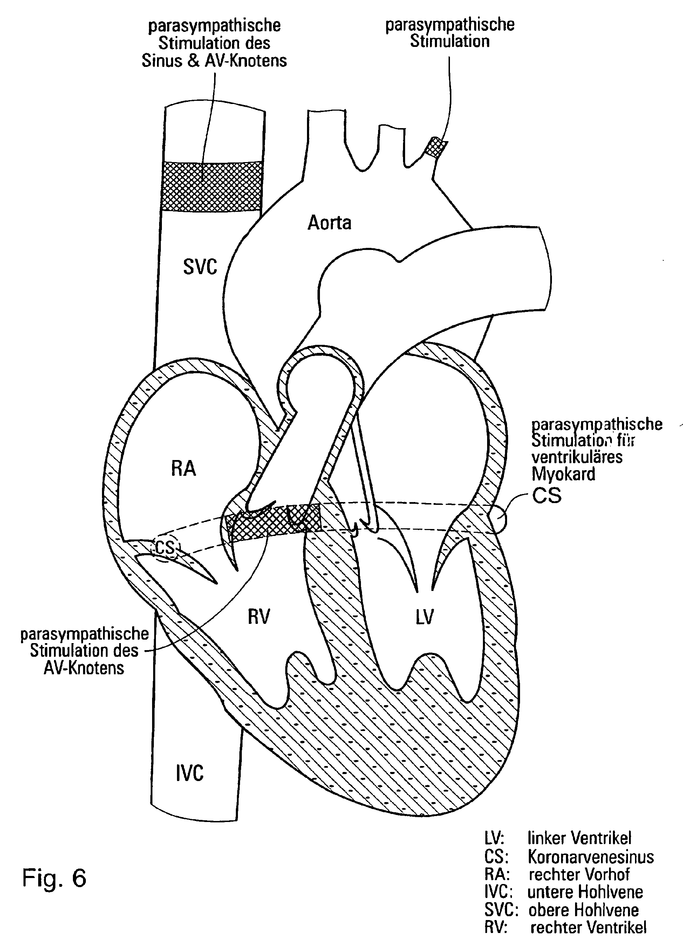

Fig. 6 gibt eine schematische Übersicht über die Anbringungsorte der genannten Elektroden im Herzbereich. Es handelt sich um eine schematische Schnittdarstellung.

Kommt es während sympathischer Stimulation zur Auslösung von gehäuften ventrikulären Extrasystolen, wird die sympathische Stimulationsintensität soweit reduziert als der arterielle Blutdruck/Herzzeitvolumen oberhalb des entsprechenden programmierten Grenzwertes bleibt. Persistieren die ventrikulären Extrasystolen, erfolgt die Initiierung einer parasympathischen Stimulation im Koronarsinus, den lugularvenen oder den oberen Hohlvenen bis die ventrikulären Extrasystolen/nicht anhaltenden Tachykardien einen programmierten Grenzwert unterschreiben. Die Intensität der parasympathischen Stimulation im Koronarsinus wird nur soweit gesteigert als es dadurch zu keiner Unterschreitung des arteriellen Blutdrucks/Herzzeitvolumens unter den programmierten Grenzwert kommt.

Fig. 5 illustriert ein Beispiel der sympathischen Stimulation der ansa subclavia zur Verhinderung einer neurokardiogenen Synkope. Typischerweise wird das Gerät durch den Patienten bei Beginn einer durch arterielle Hypotonie und/oder Bradykardie ausgelösten Schwindel-/Päsynkopen-symptomatik aktiviert und nach Besserung der Symptomatik inaktiviert. Aber auch eine automatische sympathische Stimulation im Falle eines Abfalls der Herzfrequenz und/oder des arteriellen Blutdrucks unter einen programmierten Grenzwert ist vorgesehen.

Die Impulserzeugungseinheit kann aus einem implantierbaren Generator bestehen, der in direktem elektrischen und/oder magnetischen Kontakt zu den Stimulations- und Sensorelektroden steht. Darüber hinaus kann es sich bei der Impulserzeugungseinheit jedoch auch um eine externe, von außen auf die Körperoberfläche aufgelegten Generator handeln, der ohne eine direkte elektrisch und/oder magnetische Verbindung zu den Stimulations- und Meßsonden zu besitzen z.B. induktiv eine Stimulationsspannung an den Elektroden erzeugen kann.

Das Gerät zur Regulation der Herzfrequenz und der Herzpumpkraft inkorporiert in seiner typischen Ausführung einen antibradykarden, ventrikulären Schrittmacher und einen antitachykarden ventrikulären implantierbaren Defibrillator. Dies ermöglicht eine antibradykarde Sicherheitsstimulation, falls es unter parasympathischer Stimulation zu einem unerwünscht starken Herzfrequenzabfall kommt. Sollte es während sympathischer Stimulation zur Induktion einer ventrikulären Tachykardie oder Kammerflimmern kommen, besteht ebenfalls ein Defibrillationsschutz.

In spezifisch modifizierten Geräten kann aber auch die Möglichkeit zur elektrischen atrialen Stimualation bzw. Defibrillation/Kardiversion implementiert sein.

Obwohl die beschriebene Erfindung sich auf ein Gerät bezieht, das eine autonome Nervenstimulation an allen beschriebenen Stimulationsorten gleichzeitig durchführen kann, sind Geräte, die sich nur eines der beschriebenen Nervenstimulationsverfahren bedienen, ebenfalls durch die vorliegende Erfindung angesprochen. Grundsätzlich kann ein Gerät jedes der verschiedenen Neurostimulationsverfahren alleine oder in Kombination mit anderen Neurostimulationsverfahren durchführen. Auch die Zahl der Sensoren der Herzfrequenz oder anderer biologischer Parameter kann variieren. Schließlich ist auch die Kombination des Neurostimulators mit einer Vielzahl von herkömmlichen, antibradykarden und/oder antitachykarden Schrittmachertypen/Defibrillatoren angesprochen.

## Patentansprüche

1. Gerät zur Therapie supraventrikulärer und ventrikulärer bradykarder und tachykarder Rythmusstörungen und/oder zur Beeinflussung der Herzpumpkraft umfassend
- Elektroden zur elektrischen und/oder magnetische Stimulation sympathischer und/oder parasymphatischer Nerven, die den Sinusknoten, die Vorhöfe, den atrioventrikulären Knoten oder die Herzhauptkammern innervieren mit einer zugehörigen ersten bzw. zweiten Einrichtung zur Erzeugung von elektrischen und/oder magnetischen Stimulationsimpulsen, die zu den entsprechenden Elektroden geleitet werden, wobei diese erste bzw. zweite Einrichtung durch eine zugeordnete erste bzw. zweite Einrichtung zur Erfassung eines ersten bzw. zweiten Eingangssignals aktiviert werden, wenn dieses erste bzw. zweite Eingangssignal einem vorgegebenen Kriterium entsprechen,
- Elektroden zur elektrischen und/oder magnetischen Stimulation der Atrien und Ventrikel und/oder zur ventrikulären Kardioversion/Defibrillation mit einer zugehörigen dritten bzw. vierten Einrichtung zur Erzeugung von elektrischen und/oder magnetischen Stimulationsimpulsen, die zu diesen Elektroden geleitet werden, wobei diese dritte bzw. vierte Einrichtung durch eine zugeordnete dritte bzw. vierte Einrichtung zur Erfassung eines dritten bzw. vierten Eingangssignals aktiviert werden, wenn dieses dritte bzw. vierte Eingangssignal einem vorgegebenen Kriterium entsprechen,
**gekennzeichnet durch** eine Einrichtung zur Programmierung eines Regelzusammenhanges, welcher ein Maß für die Intensität der einzuleitenden sympathischen oder parasympathischen Stimualtion in Abhängigkeit von der Intensität einer ein Maß für den aktuellen Herzleistungsbedarf bildenden Größe ist.

2. Gerät nach Anspruch 1, umfassend eine Einrichtung zur Erfassung der Rate, mit der die menschlichen Atrien und Ventrikel schlagen, wobei diese Einrichtung atriale und ventrikuläre Kontraktionen mißt.

3. Gerät nach Anspruch 1 oder 2, umfassend eine Einrichtung zur Erfassung biologischer Parameter, wie des arteriellen Blutdruckes, des rechts- oder linksventrikulären Druckes, der Sauerstoffsättigung des Blutes oder des Herzzeitvolumens.

4. Gerät nach Anspruch 1 bis 3, umfassend je eine Einrichtung zur Programmierung von Frequenzgrenzen, oberhalb oder unterhalb der eine Schlagrate des Herzens als Tachykardie oder Bradykardie erkannt wird.

5. Geräte nach einem der Ansprüche 1 bis 4, umfassend je eine Einrichtung zur Programmierung einer Blutdruck- und/oder Herzzeitvolumengrenze, unterhalb der eine behandlungsbedürftige Herzinsuffizienz erkannt wird.

6. Gerät nach einem der vorangehenden Ansprüche, umfassend je eine Einrichtung zur Programmierung von Grenzen, oberhalb oder unterhalb der ein physiologischer Parameter eine sympathischen oder parasymathische Stimulation hervorruft.

7. Gerät nach einem der Ansprüche 1 bis 6, umfassend eine auf die Erfassungseinheit reagierende Starteinheit, die die entsprechende Stimulationsimpuls erzeugende Einrichtung aktiviert, wenn die erfaßte Herzschlagrate der Vorhöfe oder Herzhauptkammern die programmierte Frequenzgrenze überschreitet/unterschreitet.

8. Gerät nach einem der Ansprüche 1 bis 7, umfassend eine auf die Erfassungseinheit reagierende Starteinheit, die die entsprechende Stimulationsimpulse erzeugende Einrichtung aktiviert, wenn der arterielle Blutdruck und/oder das Herzzeitvolumen eine programmierte untere Grenze unterschreitet.

9. Gerät nach einem der Ansprüche 1 bis 8, umfassend eine Einheit, die die während der Stimulation die von den Erfassungseinheiten gemessene Vorhof/Herzkammerfrequenz sowie den arteriellen Blutdruck und/oder das Herzzeitvolumen mit den korrespondierenden Werten vor/ohne Stimulation und/oder den entsprechenden programmierten Grenzwerten vergleicht.

10. Gerät nach einem der Ansprüche 1 bis 9, umfassend eine Stimulationseinheit, zur atrialen und/oder ventrikulären elektrischen und/oder magnetischen myokardialen Stimulation und/oder Kardioversion/Defibrillilation.

## Claims

1. A device for treating supraventricular and ventricular bradycardic and tachycardic arrhythmias and/or for influencing the heart pumping force, comprising
- electrodes for the electrical and/or magnetic stimulation of sympathetic and/or parasympathetic nerves, which innervate the sinus node, the atria, the atrioventricular node or the main ventricles with an associated first and second device for generating electrical and/or magnetic stimulation pulses which are transmitted to the corresponding electrodes, wherein this first and second device are activated by an assigned first and second device, respectively, for detecting a first and second input signal respectively, when this first and second input signal, respectively, meet a predetermined criterion,
- electrodes for the electrical and/or magnetic stimulation of the atria and ventricles and/or for ventricular cardioversion/defibrillation with an associated third and fourth device for generating electrical and/or magnetic stimulation pulses which are conducted to these electrodes, wherein this third and fourth device are activated by an associated third and fourth device, respectively, for detecting a third and fourth input signal, respectively, when this third and fourth input signal meet a predetermined criterion, **characterised by** a device for programming a relationship of rules which is a measure of the intensity of the sympathetic or parasympathetic stimulation as a function of the intensity of a value representing a measure of the current cardiac output requirement.

2. The device according to Claim 1, comprising a device for recording the rate at which the human atria and ventricles beat, wherein this device measures atrial and ventricular contractions.

3. The device according to Claim 1 or 2, comprising a device for recording biological parameters such as arterial blood pressure, right or left ventricular pressure, oxygen saturation of blood or heart time volume.

4. Devices according to Claims 1 to 3, each comprising a device for programming frequency limits above or below which a heart rate is detected as tachycardia or bradycardia.

5. Devices according to one of Claims 1 to 4, each comprising a device for programming a blood pressure and/or heart time volume limit below which a cardiac insufficiency requiring treatment is detected.

6. Devices according to one of the preceding claims, each device comprising a device for programming limits above or below which a physiological parameter gives rise to sympathetic or parasympathetic stimulation.

7. The device according to one Claims 1 to 6, comprising a starting unit which reacts to the recording unit and which activates the device generating the corresponding stimulation pulse when the detected heart rate of the atria or main ventricles exceeds/drops below the programmed frequency limit.

8. The device according to one of Claims 1 to 7, comprising a starting unit which reacts to the recording unit and which activates the device generating the corresponding stimulation pulses when the arterial blood pressure and/or the heart time volume drops below a programmed lower level.

9. The device according to one of Claims 1 to 8, comprising a unit which compares the atrial/ventricular frequency measured by the recording units and the arterial blood pressure and/or the atrial/ventricular frequency with the corresponding values before/without stimulation and/or the corresponding programmed limit values.

10. The device according to one of Claims 1 to 9, comprising a stimulation unit for the atrial and/or ventricular electrical and/or magnetic myocardial stimulation and/or cardioversion/defibrillation.

## Revendications

1. Appareil pour la thérapie de troubles du rythme bradycardiques et tachycardiques, supraventriculaires et ventriculaires, et/ou pour influencer l'énergie de pompage du coeur comprenant
- des électrodes pour la stimulation électrique et/ou magnétique de nerfs sympathiques et/ou parasympathiques, qui innervent le noeud sinusal, les oreillettes, le noeud atrioventriculaire ou les ventricules principaux avec un premier ou un second dispositif spécifique pour générer des impulsions de stimulation électriques et/ou magnétiques, qui sont acheminées aux électrodes correspondantes, ce premier ou second dispositif étant activé par un premier ou un second dispositif associé pour l'enregistrement d'un premier ou d'un second signal d'entrée, lorsque ce premier ou second signal d'entrée correspond à un critère prédéfini,
- des électrodes pour la stimulation électrique et/ou magnétique des oreillettes et ventricules et/ou pour la cardioversion/défibrillation ventriculaire avec un troisième ou quatrième dispositif spécifique pour générer des impulsions de stimulation électriques et/ou magnétiques, qui sont acheminées à ces électrodes, ce troisième ou quatrième dispositif étant activé par un troisième ou quatrième dispositif associé pour l'enregistrement d'un troisième ou quatrième signal d'entrée lorsque ce troisième ou quatrième signal d'entrée correspond à un critère prédéfini,
**caractérisé par** un dispositif pour la programmation d'un contexte de réglage, qui est une référence pour l'intensité de la stimulation sympathique ou parasympathique à enclencher en fonction de l'intensité d'une grandeur formant une référence pour les besoins actuels en débit cardiaque.

2. Appareil selon la revendication 1, comprenant un dispositif pour l'enregistrement de la fréquence, avec laquelle les oreillettes et ventricules humains battent, ce dispositif mesurant des contractions auriculaires et ventriculaires.

3. Appareil selon la revendication 1 ou 2, comprenant un dispositif pour l'enregistrement de paramètres biologiques, comme la tension artérielle, la pression du ventricule droit ou gauche, la saturation en oxygène du sang ou du volume de temps cardiaque.

4. Appareil selon les revendications 1 à 3, comprenant chacun un dispositif pour la programmation de limites de fréquence, au-dessus ou au-dessous desquels une fréquence de battement du coeur est identifiée comme tachycardie ou bradycardie.

5. Appareils selon l'une quelconque des revendications 1 à 4, comprenant chacun un dispositif pour la programmation d'une limite de tension et/ou de volume de temps cardiaque, au-dessous de laquelle une insuffisance cardiaque nécessitant un traitement est identifiée.

6. Appareil selon l'une quelconque des revendications précédentes, comprenant un dispositif pour la programmation de limites, au-dessus ou au-dessous desquelles un paramètre physiologique entraîne une stimulation sympathique ou parasympathique.

7. Appareil selon l'une quelconque des revendications 1 à 6, comprenant une unité de démarrage réagissant à l'unité d'enregistrement, qui active le dispositif générant des impulsions de stimulation appropriées lorsque le taux de battement du coeur enregistré des oreillettes ou des ventricules principaux dépasse ou sous-dépasse la limite de fréquence programmée.

8. Appareil selon l'une quelconque des revendications 1 à 7, comprenant une unité de démarrage réagissant à l'unité d'enregistrement, qui active le dispositif générant des impulsions de stimulation appropriées lorsque la tension artérielle et/ou le volume de temps cardiaque sous-dépasse une limité inférieure programmée.

9. Appareil selon l'une quelconque des revendications 1 à 8, comprenant une unité qui compare pendant la stimulation la fréquence d'oreillette/de ventricule mesurée par les unités d'enregistrement ainsi que la tension artérielle et/ou le volume de temps cardiaque avec les valeurs correspondantes avant/sans stimulation et/ou avec les valeurs limites programmées correspondantes.

10. Appareil selon l'une quelconque des revendications des revendications 1 à 9, comprenant un dispositif de stimulation, pour la stimulation atriale et/ou ventriculaire, myocardiaque électrique et/ou magnétique et/ou la cardioversion/défibrillation.
